# EUROPEAN PATENT APPLICATION

(11) **EP 3 354 634 A1**
(43) Date of publication of application: **01.08.2018**
(21) Application number: 17386001.6
(22) Date of filing: 30.01.2017
(51) Int. Cl.: C07C 5/48, C07C 11/04

(54) **ETHANE OXIDATIVE DEHYDROGENATION**

(71) Applicant: Shell Internationale Research Maatschappij B.V., 2596 HR The Hague (NL)
(72) Inventor: Mitkidis, Georgios, 1031HW Amsterdam (NL); Peltier, Todd Paul, Houston, Texas 77094 (US); Rossum VAN, Guus, 1031 HW Amsterdam (NL)
(74) Representative: Shell Legal Services IP

(57) **Abstract**

The present invention relates to a process for oxidative dehydrogenation of ethane, comprising the steps of: (a) contacting, in a reactor, oxygen and ethane with a catalyst comprising a mixed metal oxide, resulting in a reactor effluent comprising ethylene, ethane, carbon dioxide, water and acetic acid; (b) cooling the reactor effluent to obtain a liquid stream comprising water and acetic acid and a gas stream comprising ethylene, ethane and carbon dioxide; (c) removing carbon dioxide from at least a part of the gas stream comprising ethylene, ethane and carbon dioxide resulting in a gas stream comprising ethylene and ethane; (d) passing at least a part of the gas stream comprising ethylene and ethane as obtained in step (c) through a membrane, preferably a membrane comprising metal cations, more preferably a membrane comprising silver(I) ions (Ag⁺ ions) or copper(I) ions (Cu⁺ ions), to obtain a stream comprising ethane and a stream comprising ethylene.

## Description

### Field of the invention

The present invention relates to a process for oxidative dehydrogenation (oxydehydrogenation; ODH) of ethane into ethylene.

### Background of the invention

It is known to oxidatively dehydrogenate ethane resulting in ethylene, respectively, in an oxidative dehydrogenation (oxydehydrogenation; ODH) process. Examples of ethane ODH processes, including catalysts and other process conditions, are for example disclosed in US7091377, WO2003064035, US20040147393, WO2010096909, US20100256432 and CA1262556. Mixed metal oxide catalysts, for example mixed metal oxide catalysts containing molybdenum (Mo) and vanadium (V) and optionally other metals can be used as oxydehydrogenation catalysts.

In a process for oxidative dehydrogenation of ethane into ethylene, carbon dioxide, water and acetic acid are also formed. After removing said carbon dioxide, water and acetic acid, a stream comprising ethylene and unconverted ethane may be purified to obtain ethylene having a high purity.

It is an objective of the present invention to provide an improved process for ethane oxidative dehydrogenation. One aim is to obtain relatively pure ethylene. Another aim is to reduce the energy required for purifying ethylene.

### Summary of the invention

Surprisingly it was found that the above-mentioned objectives may be obtained by means of the ethane ODH process of the present invention.

The present invention relates to a process for oxidative dehydrogenation of ethane, comprising the steps of:
(a) contacting, in a reactor, oxygen and ethane with a catalyst comprising a mixed metal oxide, resulting in a reactor effluent comprising ethylene, ethane, carbon dioxide, water and acetic acid;
(b) cooling the reactor effluent to obtain a liquid stream comprising water and acetic acid and a gas stream comprising ethylene, ethane and carbon dioxide;
(c) removing carbon dioxide from at least a part of the gas stream comprising ethylene, ethane and carbon dioxide resulting in a gas stream comprising ethylene and ethane;
(d) passing at least a part of the gas stream comprising ethylene and ethane as obtained in step (c) through a membrane, preferably a membrane comprising metal cations, more preferably a membrane comprising silver(I) ions (Ag⁺ ions) or copper (I) ions (Cu⁺ ions), to obtain a stream comprising ethane and a stream comprising ethylene.

In the process of the present invention additional steps may be present. For example, use may be made of one or more additional membranes. Additionally or alternatively, a distillation step may be added. Additionally or alternatively, use may be made of a Pressure Swing Adsorption (PSA) unit.

With the ethane ODH process of the present invention, especially with the preferred embodiments that will be discussed below, it is possible to obtain a high yield of relative pure ethylene at a reduced energy demand.

### Brief description of the drawings

Figure 1 is a schematic drawing of a process for oxidative dehydrogenation of ethane in accordance with one embodiment of the present invention.
Figure 2 is a schematic drawing of a part of a preferred process in which two membranes and a distillation column are used.
Figure 3 is a schematic drawing of a part of a preferred process in which one membrane and a PSA unit are used.
Figure 4 is a schematic drawing of a part of a preferred embodiment in which two membranes and a PSA unit are used.
Figure 5 is a schematic drawing of a part of a preferred process in which one membrane, a PSA unit, and a distillation column are used.
Figure 6 is a schematic drawing of a part of a preferred process in which a distillation column, a membrane and a PSA unit are used.

### Detailed description of the invention

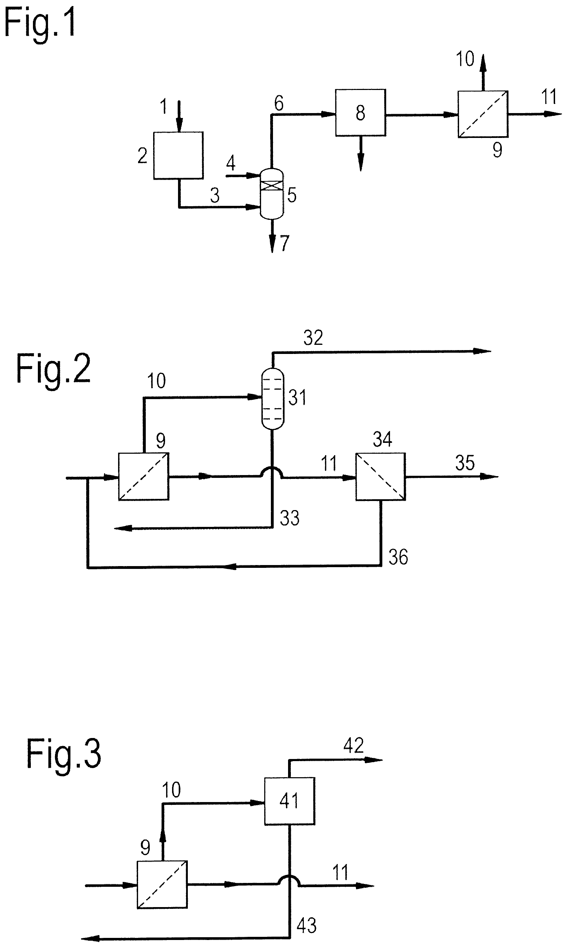

The present invention relates to a process for oxidative dehydrogenation of ethane according to claim 1. A schematic drawing of a process for oxidative dehydrogenation of ethane in accordance with one embodiment of the present invention is shown in Figure 1, which is described in detail in the "Figures" section.

### Step (a)

In step (a) oxygen and ethane are contacted with a catalyst in a reactor, preferably an ODH reactor.

In step (a), an ODH reactor may, for example, be a fluidized bed or a fixed bed reactor. In case of a fixed bed reactor it may be a (multi-) tubular fixed bed reactor. An ODH reactor of a fixed bed type can comprise, for example, catalyst pellets or catalyst extrudates.

In one embodiment there additionally may be ethylene present in a recycle stream to the reactor. Especially a recycle stream comprising ethane may also comprise ethylene.

In one embodiment, a gas stream comprising ethane and another gas stream comprising oxygen are fed to the reactor. In another embodiment, a gas stream comprising ethane and oxygen are fed to the reactor.

The one gas stream or multiple gas streams which are fed to the ODH reactor may additionally comprise an inert gas. An inert gas is defined as a gas that does not take part in the reaction of ethane and optionally ethylene with oxygen. The inert gas may be selected from the group consisting of the noble gases and nitrogen (N₂). Preferably, the inert gas is nitrogen or argon, more preferably nitrogen. In case air is fed to the reactor, one or multiple gas streams comprise oxygen as well as nitrogen.

Suitable molar ratios of oxygen to ethane fed to the reactor are in the range of from 0.01 to 3, more suitably 0.05 to 1, even more suitably 0.1 to 0.5. In case more than one gas stream is fed to the reactor a combined gas stream is formed inside the reactor. In a preferred embodiment, the (combined) gas comprises 5 to 40 vol.% of oxygen, more suitably 15 to 35 vol.% of oxygen, and 40 to 80 vol.% of ethane, more suitably 50 to 70 vol.% of ethane. The (combined) gas preferably comprises no or less than 80 vol.%, i.e. 0 to 80 vol%, of inert gas, more suitably less than 50 (0 to 50) vol.% of inert gas, more suitably 5 to 35 vol.% of inert gas, most suitably 10 to 20 vol.% of inert gas. The inert gas may, for example, be nitrogen. In the context of the present invention, the components of said gas stream are to be selected in an overall amount not to exceed 100 vol.%.

The ratio of oxygen to the ethane and the volume percentages for oxygen, ethane and inert gas are the ratio and volume percentages, respectively, at the entrance of the catalyst bed. Obviously, after entering the catalyst bed, at least part of the oxygen and ethane from the gas stream gets consumed.

Examples of oxydehydrogenation processes, including catalysts and other process conditions, are for example disclosed in above-mentioned US7091377, WO2003064035, US20040147393, WO2010096909 and US20100256432, the disclosures of which are herein incorporated by reference.

The amount of the catalyst in said process is not essential. Preferably, a catalytically effective amount of the catalyst is used, that is to say an amount sufficient to promote an oxidative dehydrogenation reaction in step (a).

In step (a) of the process of the present invention, the gas hourly space velocity (GHSV; in m³ gas/m³ catalyst/hr) may typically be of from 100 to 50,000 hr⁻¹. Said GHSV is measured at standard temperature and pressure, namely 32 °F (0 °C) and 1 bara (100 kPa). In a preferred embodiment of the present invention, said GHSV is of from 2,500 to 25,000 hr⁻¹, more preferably of from 5,000 to 20,000 hr⁻¹, most preferably of from 7,500 to 15,000 hr⁻¹.

In step (a) of the process of the present invention, typical pressures are 0.1-20 bara (i.e. "bar absolute"), and typical temperatures are 100-600 °C, suitably 200-500 °C. Step (a) preferably is performed at a temperature in the range of from 300 to 500 °C, more preferably of from 310 to 450 °C, more preferably of from 320 to 420 °C, most preferably of from 330 to 420 °C.

Further, in a preferred embodiment of the present invention, the pressure in step (a) is in the range of from 0.1 to 15 bara, more preferably of from 0.5 to 10 bara, even more preferably of from 1 to 10 bara. Step (a) still more preferably is performed at a pressure in the range of from 1.5 to 6 bara.

The catalyst used in step (a) comprises a mixed metal oxide. The catalyst preferably comprises 35 to 99.9 wt%, more preferably 45 to 99.9 wt%, even more preferably 75 to 99.9 wt%, of said mixed metal oxide.

In the present invention, said mixed metal oxide may be a mixed metal oxide of molybdenum, vanadium, niobium and optionally tellurium which may have the following formula:

Mo₁VₐTe_{b}Nb_{c}Oₙ

wherein:
a, b, c and n represent the ratio of the molar amount of the element in question to the molar amount of molybdenum (Mo);
a (for V) is from 0.01 to 1, preferably 0.05 to 0.60, more preferably 0.10 to 0.40, more preferably 0.20 to 0.35, most preferably 0.25 to 0.30;
b (for Te) is 0 or from >0 to 1, preferably 0.01 to 0.40, more preferably 0.05 to 0.30, more preferably 0.05 to 0.20, most preferably 0.09 to 0.15;
c (for Nb) is from >0 to 1, preferably 0.01 to 0.40, more preferably 0.05 to 0.30, more preferably 0.10 to 0.25, most preferably 0.14 to 0.20; and
n (for O) is a number which is determined by the valency and frequency of elements other than oxygen

Further, in the present invention, said mixed metal oxide may comprise:
- molybdenum, vanadium and antimony, or
- molybdenum, vanadium, niobium and optionally tellurium or antimony.

Still further, in the present invention, said mixed metal oxide may comprise:
- molybdenum, vanadium and antimony, or
- molybdenum, vanadium, niobium and tellurium, or
- molybdenum, vanadium, niobium and antimony.

Suitable preparation methods for such mixed metal oxides are known to a person skilled in the art. Suitable preparation methods are, for example, described in WO2015082598, US5534650, Manuel Baca et al., Applied Catalysis A: General 279, pages 67-77, 2005; W.D. Pyrz et al., PNAS, vol 107, no. 14, April 2010 and the Supporting Information: Pyrz et al. 10.1073/pnas. 1001239107; E.K. Novakova et al., Journal of Catalysis 211, pages 226-234, 2002.

The catalyst used in step (a) is a particulate catalyst, preferably a heterogeneous catalyst in the form of particles. The particles may be of any size suitable to be used in the reactor. The particles may be small enough to be used in a fluidized bed reactor. Alternatively, the particles may be arranged in a catalyst bed in the reactor. In that case the reactor may be a (multi-) tubular fixed bed reactor. Such a catalyst bed may comprise pellets, extrudates, or catalyst on a metal support (like a metal wire or metal flake). In addition to catalyst particles, the catalyst bed may also contain inert particles, i.e. catalytically inactive particles.

During step (a), ethylene and water are formed by oxidative dehydrogenation of ethane. Further, acetic acid is formed by oxidation of ethylene. Still further, carbon dioxide is formed as a by-product. During step (a) gas is fed to the reactor and an effluent is withdrawn from the reactor. The reactor effluent comprises ethylene, ethane, carbon dioxide, water and acetic acid. Said ethane comprises unconverted ethane.

### Step (b)

In step (b) the reactor effluent is cooled. Simultaneously and/or in a subsequent step, water may be added. In step (b), by cooling the reactor effluent, a liquid stream comprising water and acetic acid and a gas stream comprising ethylene, ethane and carbon dioxide are obtained.

Preferably water is added to the reactor effluent, for example by means of a water-wash scrubber. Most preferably almost all acetic acid is washed out of the reactor effluent by said additional water.

### Step (c)

In step (c) carbon dioxide is removed from at least a part of the gas stream comprising ethylene, ethane and carbon dioxide, resulting in a gas stream comprising ethylene and ethane. This is preferably performed using one or more amines and/or by means of caustic treating. Caustic treating may be performed, for example, using a sodium hydroxide solution. A suitable carbon dioxide removal agent may be an aqueous solution of a base, for example sodium hydroxide or an amine.

### Step (d)

In step (d) at least a part of the gas stream comprising ethylene and ethane as obtained in step (c) is passed through a membrane. A stream comprising ethane and a stream comprising ethylene are obtained in step (d). Optionally, at least a part of the stream comprising ethane as obtained in step (d) may be fed to the reactor used in step (a).

At least a part of the gas stream comprising ethylene and ethane as obtained in step (c) may be subjected to distillation, to remove ethane, before it is passed through the membrane in step (d). Preferably, before carrying out said distillation, any water is removed from at least a part of the gas stream comprising ethylene and ethane as obtained in step (c). Said water may be residual water originating from the carbon dioxide removal agent used in step (c). Said water may for example be removed by means of molecular sieves or glycol dehydration. Said distillation step results in a top stream comprising ethylene and ethane and a bottom stream comprising ethane. Said top stream is sent to step (d).

Preferably the membrane used in step (d) comprises metal cations, more preferably transition metal cations. Any membrane comprising metal cations, capable of separating ethylene from ethane, may be used. Ethylene may preferentially complex to said metal cations. For example, the membrane may comprise one or more metal cations selected from the group consisting of silver(I) ions (Ag⁺ ions), copper(I) ions (Cu⁺ ions), gold(I) ions (Au⁺ ions), nickel(II) ions (Ni²⁺ ions), platinum(II) ions (Pt²⁺ ions), palladium(II) ions (Pd²⁺ ions). Suitably, the membrane comprises silver(I) ions or copper(I) ions, more suitably silver(I) ions.

Suitable membranes which may be used in step (d) are for example available from Imtex (Innovative Membrane Technologies) and CMS (Compact Membrane Systems). Suitable membranes which may be used in step (d) are membranes as described in WO2004096415, WO2012167362, WO201080753 and CA2918287, the disclosures of which are herein incorporated by reference.

Suitable membranes which may be used in step (d) comprise a supporting substrate and additionally a layer comprising the above-mentioned metal cations. Any supporting substrate may be used. For example, the supporting substrate may comprise a polyester, polyamide, polyimide, polyacrylonitrile, polysulphone, polycarbonate, or a combination thereof. Further, said layer comprising the above-mentioned metal cations may be composed of any material. For example, said cations containing layer may be a polysaccharide layer, in particular a chitosan layer.

A further suitable membrane which may be used in step (d) comprises a nonporous, selectively permeable layer of a copolymer comprising copolymerized perfluorinated cyclic or cyclizable monomer, and a 4-carbon acid/anhydride, wherein the copolymer may further comprise an acyclic fluorinated olefinic monomer. In particular, a metal exchanged fluorinated ionomer may be used which is a copolymer minimally including repeating units of (i) a polymerized derivative of a perfluorinated cyclic or cyclizable monomer and (ii) a strong acid highly fluorinated vinylether compound in which the acid moiety is exchanged with a cation of a Group 11 metal. Metal exchanged fluorinated ionomers are readily soluble and can be formed into thin, selectively gas permeable membranes by solution deposition methods.

The membrane used in step (d) preferably is operated at a pressure in the range of from 1 to 100 bara, preferably 5 to 50 bara, more preferably 10 to 30 bara. The membrane used in step (d) preferably is operated at a temperature in the range of from 0 to 100°C, preferably 10 to 60°C, more preferably 20 to 60°C.

In a case wherein in one of the below-described optional further steps, separation using a further membrane ("second membrane") is carried out, such further membrane separation step may be carried out in the same way as step (d), using a "first membrane", as described above.

### Optional further steps

In a case wherein step (a) is performed in the presence of methane and/or nitrogen, the process of the present invention comprises the steps of:
(a) contacting, in a reactor, oxygen and ethane with a catalyst comprising a mixed metal oxide in the presence of methane and/or nitrogen, resulting in a reactor effluent comprising ethylene, ethane, carbon dioxide, water and acetic acid and additionally methane and/or nitrogen;
(b) cooling the reactor effluent to obtain a liquid stream comprising water and acetic acid and a gas stream comprising ethylene, ethane and carbon dioxide and additionally methane and/or nitrogen;
(c) removing carbon dioxide from at least a part of the gas stream comprising ethylene, ethane and carbon dioxide and additionally methane and/or nitrogen resulting in a gas stream comprising ethylene and ethane and additionally methane and/or nitrogen;
(d) passing at least a part of the gas stream comprising ethylene and ethane and additionally methane and/or nitrogen as obtained in step (c) through a membrane, preferably a membrane comprising metal cations, more preferably a membrane comprising silver(I) ions (Ag⁺ ions) or copper(I) ions (Cu⁺ ions), to obtain a stream comprising ethane and additionally methane and/or nitrogen and a stream comprising ethylene.
Said methane may originate from the ethane feed to step (a). Further, said nitrogen may originate from air fed as oxygen containing source to step (a).

In the above-described case, wherein step (a) is performed in the presence of methane and/or nitrogen, the process of the present invention may comprise one of the following further steps.

At least part of the stream comprising ethane and additionally methane and/or nitrogen as obtained in step (d) in the above-described case may be subjected to distillation, resulting in a top stream comprising methane and/or nitrogen and a bottom stream comprising ethane. Preferably, before carrying out said distillation, any water is removed, as described above in relation to an optional distillation step preceding step (d). At least part of the stream comprising ethane as obtained in this distillation step may be fed to the reactor used in step (a). Said distillation step after step (d) is illustrated in Figure 2, as further described below.

Further, at least part of the stream comprising ethane and additionally methane and/or nitrogen as obtained in step (d) in the above-described case may be fed to a Pressure Swing Adsorption (PSA) unit to obtain a stream comprising ethane and a stream comprising methane and/or nitrogen. PSA units are commercially available. Suitable adsorbents to be used in the PSA unit are, for example, molecular sieves, zeolites, such as zeolite 13X and zeolite 5A, activated carbon, silica gel, activated alumina and crystalline titanium silicate. In the PSA unit, the pressure in a desorption step is lower than the pressure in the sorption step that directly precedes said desorption step. In case desorption is effected by reducing the pressure, the pressure in said sorption step may be the same as the pressure at which the membrane used in step (d) is operated, as described above. The temperature in the PSA unit may vary within wide ranges. Preferably, the temperature is in the range of from 0 to 110 °C, more preferably 10 to 90 °C, most preferably 25 to 80 °C. Advantageously, said step may be carried out at a non-cryogenic temperature, e.g. of from 0 to 110 °C. At least part of the stream comprising ethane as obtained in this PSA step may be fed to the reactor used in step (a). Said PSA step after step (d) is illustrated in Figures 3, 4, 5 and 6, as further described below.

Further, in a case wherein the stream comprising ethylene as obtained in step (d) also comprises ethane, one of the following further steps may be performed.

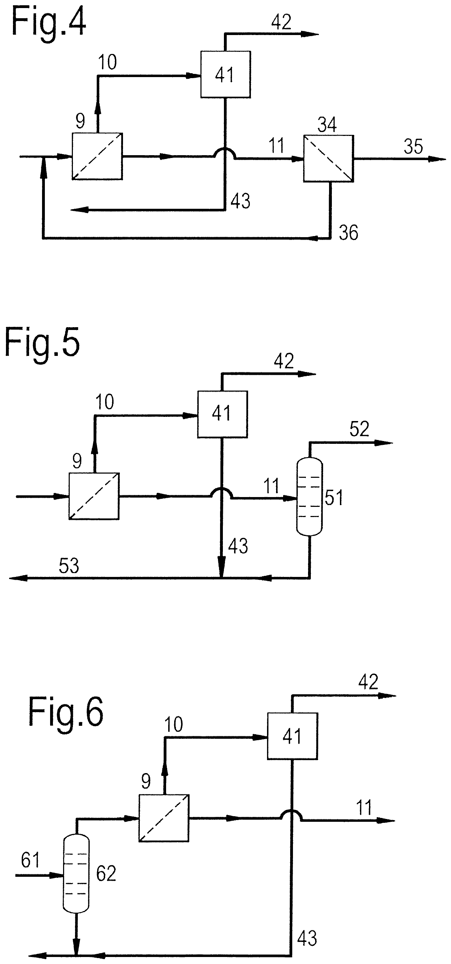

At least part of the stream comprising ethane and ethylene as obtained in step (d) in the above-described case may be subjected to distillation, resulting in a top stream comprising ethylene and a bottom stream comprising ethane. The former stream comprising ethylene may be a stream of high purity ethylene of polymer grade. Preferably, before carrying out said distillation, any water is removed, as described above in relation to an optional distillation step preceding step (d). At least part of the stream comprising ethane as obtained in this distillation step may be fed to the reactor used in step (a). Said distillation step after step (d) is illustrated in Figure 5, as further described below.

Further, at least part of the stream comprising ethane and ethylene as obtained in step (d) in the above-described case may be passed through a second membrane, preferably a membrane comprising metal cations, more preferably a membrane comprising silver(I) ions (Ag⁺ ions) or copper(I) ions (Cu⁺ ions), to obtain a stream comprising ethane and a stream comprising ethylene. The latter stream comprising ethylene may be a stream of high purity ethylene of polymer grade. At least part of the stream comprising ethane as obtained in this second membrane separation step may be fed to the reactor used in step (a). Said second membrane separation step after step (d) is illustrated in Figures 2 and 4, as further described below.

Further, more specific embodiments of the present invention are described hereinbelow.

### Two membranes and a distillation

In one preferred embodiment of the process use is made of two membranes and a distillation column. A schematic drawing of a preferred example of this embodiment is shown in Figure 2, which is described in detail in the "Figures" section. One stream obtained by means of the first membrane is fed to a distillation column. Another stream obtained by means of the first membrane is fed to a second membrane.

In this preferred embodiment, step (a) is performed in the presence of methane and/or nitrogen as in the above-described case, in step (d) a stream comprising ethane and additionally methane and/or nitrogen and a stream comprising ethylene and ethane are obtained, and step (d) is followed by the following steps:
(e) feeding the stream comprising ethane and additionally methane and/or nitrogen as obtained in step (d) to a distillation column to obtain a stream comprising ethane and a stream comprising methane and/or nitrogen;
(f) optionally feeding at least a part of the stream comprising ethane as obtained in step (e) to the reactor used in step (a);
(g) passing at least a part of the stream comprising ethylene and ethane as obtained in step (d) through a second membrane, preferably a membrane comprising metal cations, more preferably a membrane comprising comprising silver(I) ions (Ag⁺ ions) or copper(I) ions (Cu⁺ ions), to obtain a stream comprising ethane and ethylene and a stream comprising ethylene;
(h) optionally feeding at least a part of the stream comprising ethane and ethylene as obtained in step (g) to the membrane used in step (d), that is to say to the first membrane, or optionally feeding at least a part of the stream comprising ethane and ethylene as obtained in step (g) to the reactor used in step (a).

The distillation in step (e) preferably is performed at a pressure in the range of from 1 to 100 bara, preferably 10 to 50 bara, more preferably 15 to 35 bara.

Step (f) is optional. In step (f) at least a part of the stream comprising ethane as obtained in step (e) is fed to the reactor, the ODH reactor, used in step (a). This stream can be fed directly to the reactor, or it can be mixed with a stream comprising oxygen and/or a stream comprising ethane before it is fed to the reactor.

In step (g) at least a part of the stream comprising ethylene and ethane as obtained in step (d) is passed through a second membrane. Step (g) may be carried out in the same way as step (d) as described above. In particular, the second membrane used in step (g) may be the same or different as compared to the first membrane which is used in step (d).

Step (h) is optional. Step (h) is a recycle step.

### One membrane and a PSA unit

In one preferred embodiment of the process use is made of one membrane and a Pressure Swing Adsorption (PSA) unit. A schematic drawing of a preferred example of this embodiment is shown in Figure 3, which is described in detail in the "Figures" section. One stream obtained by means of the membrane is fed to a PSA unit.

In this preferred embodiment, step (a) is performed in the presence of methane and/or nitrogen as in the above-described case, and step (d) is followed by the following steps:
(eA) feeding the stream comprising ethane and additionally methane and/or nitrogen as obtained in step (d) to a Pressure Swing Adsorption (PSA) unit to obtain a stream comprising ethane and a stream comprising methane and/or nitrogen;
(fA) optionally feeding at least a part of the stream comprising ethane as obtained in step (eA) to the reactor used in step (a).

Step (eA), using a Pressure Swing Adsorption (PSA) unit, may be carried out in the same way as the PSA step as described above.

### Two membranes and a PSA unit

In one preferred embodiment of the process use is made of two membranes and a Pressure Swing Adsorption (PSA) unit. A schematic drawing of a preferred example of this embodiment is shown in Figure 4, which is described in detail in the "Figures" section.

In this preferred embodiment, step (a) is performed in the presence of methane and/or nitrogen as in the above-described case, in step (d) a stream comprising ethane and additionally methane and/or nitrogen and a stream comprising ethylene and ethane are obtained, and step (d) is followed by the following steps:
(eA) feeding the stream comprising ethane and additionally methane and/or nitrogen as obtained in step (d) to a Pressure Swing Adsorption (PSA) unit to obtain a stream comprising ethane and a stream comprising methane and/or nitrogen;
(fA) optionally feeding at least a part of the stream comprising ethane as obtained in step (eA) to the reactor used in step (a);
(gA) passing at least a part of the stream comprising ethylene and ethane as obtained in step (d) through a second membrane, preferably a membrane comprising metal cations, more preferably a membrane comprising comprising silver(I) ions (Ag⁺ ions) or copper(I) ions (Cu⁺ ions), to obtain a stream comprising ethane and ethylene and a stream comprising ethylene;
(hA) optionally feeding at least a part of the stream comprising ethane and ethylene as obtained in step (gA) to the membrane used in step (d), that is to say to the first membrane, or optionally feeding at least a part of the stream comprising ethane and ethylene as obtained in step (gA) to the reactor used in step (a).

Steps (eA) and (fA) are as described above. Step (gA) is as described for step (g) above. Step (hA) is as described for step (h) above.

### Membrane, PSA and distillation

In one preferred embodiment of the process use is made of a membrane, a Pressure Swing Adsorption (PSA) unit and a distillation column. A schematic drawing of a preferred example of this embodiment is shown in Figure 5, which is described in detail in the "Figures" section.

In this preferred embodiment, step (a) is performed in the presence of methane and/or nitrogen as in the above-described case, in step (d) a stream comprising ethane and additionally methane and/or nitrogen and a stream comprising ethylene and ethane are obtained, and step (d) is followed by the following steps:
(eA) feeding the stream comprising ethane and additionally methane and/or nitrogen as obtained in step (d) to a Pressure Swing Adsorption (PSA) unit to obtain a stream comprising ethane and a stream comprising methane and/or nitrogen;
(fA) optionally feeding at least a part of the stream comprising ethane as obtained in step (eA) to the reactor used in step (a);
(gB) feeding the stream comprising ethylene and ethane as obtained in step (d) to a distillation column to obtain a stream comprising ethane and a stream comprising ethylene;
(hB) optionally feeding at least a part of the stream comprising ethane as obtained in step (gB) to the reactor used in step (a).

Steps (eA) and (fA) are as described above. Preferably, before carrying out step (gB), any water is removed, as described above in relation to an optional distillation step preceding step (d). The distillation in step (gB) preferably is performed at a pressure in the range of from 1 to 100 bara, preferably 10 to 50 bara, more preferably 15 to 35 bara. Alternatively or additionally, step (fA) may comprise optionally feeding at least a part of the stream comprising ethane as obtained in step (eA) to distillation step (gB).

### Distillation, membrane and PSA

In one preferred embodiment of the process use is made of a distillation column, a membrane and a Pressure Swing Adsorption (PSA) unit. A schematic drawing of a preferred example of this embodiment is shown in Figure 6, which is described in detail in the "Figures" section.

In this preferred embodiment, at least a part of the gas stream comprising ethylene and ethane and additionally methane and/or nitrogen as obtained in step (c) is subjected to distillation, resulting in a stream comprising ethane and a stream comprising ethylene and ethane and additionally methane and/or nitrogen which latter stream is fed to step (d), and step (d) is followed by the following steps:
(eA) feeding the stream comprising ethane and additionally methane and/or nitrogen as obtained in step (d) to a Pressure Swing Adsorption (PSA) unit to obtain a stream comprising ethane and a stream comprising methane and/or nitrogen;
(fA) optionally feeding at least a part of the stream comprising ethane as obtained in step (eA) to the reactor used in step (a);
(gC) optionally feeding at least a part of the stream comprising ethane as obtained in the distillation to the reactor used in step (a).

Preferably, before carrying out said distillation, any water is removed, as described above in relation to an optional distillation step preceding step (d). Said distillation step results in a top stream comprising ethylene and ethane and additionally methane and/or nitrogen and a bottom stream comprising ethane. Said top stream is fed to step (d). Steps (eA) and (fA) are as described above. Alternatively or additionally, step (fA) may comprise optionally feeding at least a part of the stream comprising ethane as obtained in step (eA) to said distillation step.

### Figures

The present invention will be further elucidated with reference to the drawings.

Figure 1 is a schematic drawing of a process for oxidative dehydrogenation of ethane in accordance with one embodiment of the present invention. Gas comprising oxygen and ethane is fed (1) to a reactor (2). In the reactor (2), which is an ODH reactor, oxygen and ethane are contacted with a catalyst comprising a mixed metal oxide in the presence of methane and nitrogen. The reactor effluent comprising ethylene, ethane, carbon dioxide, water, acetic acid, methane and nitrogen is fed (3) to a separator (5). Water is fed (4) to the separator (5). The reactor effluent is cooled. A liquid stream (7) comprising water and acetic acid is separated from the gas stream. The remaining gas stream comprising ethylene, ethane, carbon dioxide, methane and nitrogen is fed (6) to carbon dioxide removal unit (8). A suitable carbon dioxide removal agent fed to said carbon dioxide removal unit (8) may be an aqueous solution of a base, for example sodium hydroxide or an amine. The gas stream comprising ethylene is passed through membrane (9). Membrane (9) may be a membrane as described above. A stream comprising ethane, methane and nitrogen (10) is obtained. And a stream comprising ethylene (11) is obtained.

The following Figures 2 to 6 elucidate further embodiments of the embodiment of Figure 1.

Figure 2 is a schematic drawing of a part of a preferred process in which two membranes and a distillation column are used. The stream comprising ethane, methane and nitrogen (10) is fed to a distillation column (31). A stream comprising methane and nitrogen (32) is obtained. A stream comprising ethane (33) is obtained which is optionally recycled to ODH reactor (2) (see Figure 1). The stream comprising ethylene (11) also comprises ethane and is passed through a second membrane (34). Second membrane (34) may be the same as first membrane (9). A stream comprising ethylene (35), which is a product stream, is obtained. A stream comprising ethane and ethylene (36) is recycled to the first membrane (9). Said stream comprising ethane and ethylene (36) may also be recycled to ODH reactor (2) (see Figure 1).

Figure 3 is a schematic drawing of a part of a preferred process in which one membrane and a PSA unit are used. The stream comprising ethane, methane and nitrogen (10) is fed to a PSA unit (41). A stream comprising methane and nitrogen (42) is obtained. And a stream comprising ethane (43) is obtained which is optionally recycled to ODH reactor (2) (see Figure 1).

Figure 4 is a schematic drawing of a part of a preferred embodiment in which two membranes and a PSA unit are used. The stream comprising ethane, methane and nitrogen (10) is fed to a PSA unit (41). A stream comprising methane and nitrogen (42) is obtained. And a stream comprising ethane (43) is obtained which is optionally recycled to ODH reactor (2) (see Figure 1). The stream comprising ethylene (11) also comprises ethane and is passed through a second membrane (34). Second membrane (34) may be the same as first membrane (9). A stream comprising ethylene (35), which is a product stream, is obtained. A stream comprising ethane and ethylene (36) is recycled to the first membrane (9). Said stream comprising ethane and ethylene (36) may also be recycled to ODH reactor (2) (see Figure 1).

Figure 5 is a schematic drawing of a part of a preferred process in which one membrane, a PSA unit and a distillation column are used. The stream comprising ethane, methane and nitrogen (10) is fed to a PSA unit (41). A stream comprising methane and nitrogen (42) is obtained. And a stream comprising ethane (43) is obtained. The stream comprising ethylene (11) also comprises ethane and is fed to a distillation column (51). A bottom stream comprising ethane and a top stream comprising ethylene (52) are obtained. The stream comprising ethane (43) may be combined with the bottom stream comprising ethane from the distillation column, and this combined stream (53) is optionally recycled to ODH reactor (2) (see Figure 1).

Figure 6 is a schematic drawing of a part of a preferred process in which a distillation column, a membrane and a PSA unit are used. A gas stream comprising ethylene, ethane, methane and nitrogen (61) originating from carbon dioxide removal unit (8) is fed to a distillation column (62). A bottom stream comprising ethane and a top stream comprising ethylene, ethane, methane and nitrogen are obtained, which top stream is fed to membrane (9). The stream comprising ethane, methane and nitrogen (10) is fed to a PSA unit (41). A stream comprising methane and nitrogen (42) is obtained. And a stream comprising ethane (43) is obtained, which may be combined with the bottom stream comprising ethane from the distillation column and this combined stream is optionally recycled to ODH reactor (2) (see Figure 1).

The present invention is also applicable to a process for oxidative dehydrogenation of alkanes having a higher carbon number than ethane, in particular alkanes having a carbon number of from 3 to 6 carbon atoms, including propane, butane, pentane and hexane, more specifically propane and butane, most specifically propane. For example, in the case of propane, step (a) of the present invention would result in a reactor effluent comprising propylene, propane, carbon dioxide, water and acrylic acid; step (b) would result in a liquid stream comprising water and acrylic acid and a gas stream comprising propylene, propane and carbon dioxide; step (c) would result in a gas stream comprising propylene and propane; and step (d) would result in a stream comprising propane and a stream comprising propylene.

## Claims

1. A process for oxidative dehydrogenation of ethane, comprising the steps of:
(a) contacting, in a reactor, oxygen and ethane with a catalyst comprising a mixed metal oxide, resulting in a reactor effluent comprising ethylene, ethane, carbon dioxide, water and acetic acid;
(b) cooling the reactor effluent to obtain a liquid stream comprising water and acetic acid and a gas stream comprising ethylene, ethane and carbon dioxide;
(c) removing carbon dioxide from at least a part of the gas stream comprising ethylene, ethane and carbon dioxide resulting in a gas stream comprising ethylene and ethane;
(d) passing at least a part of the gas stream comprising ethylene and ethane as obtained in step (c) through a membrane, preferably a membrane comprising metal cations, more preferably a membrane comprising silver(I) ions (Ag⁺ ions) or copper(I) ions (Cu⁺ ions), to obtain a stream comprising ethane and a stream comprising ethylene.

2. The process according to claim 1, comprising the steps of:
(a) contacting, in a reactor, oxygen and ethane with a catalyst comprising a mixed metal oxide in the presence of methane and/or nitrogen, resulting in a reactor effluent comprising ethylene, ethane, carbon dioxide, water and acetic acid and additionally methane and/or nitrogen;
(b) cooling the reactor effluent to obtain a liquid stream comprising water and acetic acid and a gas stream comprising ethylene, ethane and carbon dioxide and additionally methane and/or nitrogen;
(c) removing carbon dioxide from at least a part of the gas stream comprising ethylene, ethane and carbon dioxide and additionally methane and/or nitrogen resulting in a gas stream comprising ethylene and ethane and additionally methane and/or nitrogen;
(d) passing at least a part of the gas stream comprising ethylene and ethane and additionally methane and/or nitrogen as obtained in step (c) through a membrane, preferably a membrane comprising metal cations, more preferably a membrane comprising silver(I) ions (Ag⁺ ions) or copper(I) ions (Cu⁺ ions), to obtain a stream comprising ethane and additionally methane and/or nitrogen and a stream comprising ethylene.

3. The process according to claim 2, wherein in step (d) a stream comprising ethane and additionally methane and/or nitrogen and a stream comprising ethylene and ethane are obtained, and wherein step (d) is followed by:
(e) feeding the stream comprising ethane and additionally methane and/or nitrogen as obtained in step (d) to a distillation column to obtain a stream comprising ethane and a stream comprising methane and/or nitrogen;
(f) optionally feeding at least a part of the stream comprising ethane as obtained in step (e) to the reactor used in step (a);
(g) passing at least a part of the stream comprising ethylene and ethane as obtained in step (d) through a second membrane, preferably a membrane comprising metal cations, more preferably a membrane comprising comprising silver(I) ions (Ag⁺ ions) or copper(I) ions (Cu⁺ ions), to obtain a stream comprising ethane and ethylene and a stream comprising ethylene;
(h) optionally feeding at least a part of the stream comprising ethane and ethylene as obtained in step (g) to the membrane used in step (d), that is to say to the first membrane, or optionally feeding at least a part of the stream comprising ethane and ethylene as obtained in step (g) to the reactor used in step (a).

4. The process according to claim 2, wherein step (d) is followed by:
(eA) feeding the stream comprising ethane and additionally methane and/or nitrogen as obtained in step (d) to a Pressure Swing Adsorption (PSA) unit to obtain a stream comprising ethane and a stream comprising methane and/or nitrogen;
(fA) optionally feeding at least a part of the stream comprising ethane as obtained in step (eA) to the reactor used in step (a).

5. The process according to claim 4, wherein in step (d) a stream comprising ethane and additionally methane and/or nitrogen and a stream comprising ethylene and ethane are obtained, and wherein step (d) is further followed by:
(gA) passing at least a part of the stream comprising ethylene and ethane as obtained in step (d) through a second membrane, preferably a membrane comprising metal cations, more preferably a membrane comprising comprising silver(I) ions (Ag⁺ ions) or copper(I) ions (Cu⁺ ions), to obtain a stream comprising ethane and ethylene and a stream comprising ethylene;
(hA) optionally feeding at least a part of the stream comprising ethane and ethylene as obtained in step (gA) to the membrane used in step (d), that is to say to the first membrane, or optionally feeding at least a part of the stream comprising ethane and ethylene as obtained in step (gA) to the reactor used in step (a).

6. The process according to claim 4, wherein in step (d) a stream comprising ethane and additionally methane and/or nitrogen and a stream comprising ethylene and ethane are obtained, and wherein step (d) is further followed by:
(gB) feeding the stream comprising ethylene and ethane as obtained in step (d) to a distillation column to obtain a stream comprising ethane and a stream comprising ethylene;
(hB) optionally feeding at least a part of the stream comprising ethane as obtained in step (gB) to the reactor used in step (a).

7. The process according to claim 2, wherein at least a part of the gas stream comprising ethylene and ethane and additionally methane and/or nitrogen as obtained in step (c) is subjected to distillation, resulting in a stream comprising ethane and a stream comprising ethylene and ethane and additionally methane and/or nitrogen which latter stream is fed to step (d), and wherein step (d) is further followed by:
(eA) feeding the stream comprising ethane and additionally methane and/or nitrogen as obtained in step (d) to a Pressure Swing Adsorption (PSA) unit to obtain a stream comprising ethane and a stream comprising methane and/or nitrogen;
(fA) optionally feeding at least a part of the stream comprising ethane as obtained in step (eA) to the reactor used in step (a);
(gC) optionally feeding at least a part of the stream comprising ethane as obtained in the distillation to the reactor used in step (a).
